# EUROPÄISCHE PATENTSCHRIFT

(11) **EP 1 414 374 B1**
(45) Veröffentlichungstag und Bekanntmachung des Hinweises auf die Patenterteilung: **26.10.2005**
(21) Anmeldenummer: 02754493.1
(22) Anmeldetag: 07.08.2002
(51) Int. Cl.: A61F 2/06, A61L 31/18

(54) **MAGNETRESONANZKOMPATIBLE METALLISCHE ENDOPROTHESE**
METALLIC ENDOPROSTHESIS COMPATIBLE WITH MAGNETIC RESONANCE
ENDOPROTHESE METALLIQUE COMPATIBLE AVEC LA RESONANCE MAGNETIQUE

(30) Priorität: 08.08.2001 DE 20112762 U; 14.12.2001 DE 20120222 U; 03.02.2002 DE 20201637 U
(43) Veröffentlichungstag der Anmeldung: 06.05.2004
(73) Patentinhaber: Bücker, Arno, 52349 Düren (DE); Rübben, Alexander, 52074 Aachen (DE)
(72) Erfinder: Bücker, Arno, 52349 Düren (DE); Rübben, Alexander, 52074 Aachen (DE)
(74) Vertreter: Theobald, Andreas
(86) Internationale Anmeldenummer: PCT/DE2002/002903
(87) Internationale Veröffentlichungsnummer: WO 2003/015662

(56) Entgegenhaltungen:
- EP-A- 1 046 722
- WO-A-01/37761
- WO-A-99/44543
- DE-C- 19 811 033

## Beschreibung

Die vorliegende Erfindung betrifft eine Endoprothese, die MR-kompatibel ist.

Endoprothesen werden derzeit zum Beispiel im Bereich der Gefässe (arteriell und venös), der Gallenwege, der Luftwege sowie des Magen-Darm-Traktes eingesetzt, um Hohlräume offen zu halten. Die unterschiedlichen Indikationen haben zur Entwicklung verschiedener Typen von Endoprothesen hinsichtlich ihres Designs und ihrer Form geführt. Des weiteren werden die Endoprothesen aus verschiedensten Materialien hergestellt. Insbesondere für die intravasale Anwendung von Endoprothesen - sogenannten Stents - hat sich bisher Metall als das am besten geeignete Material erwiesen. Dementsprechend wird die überwiegende Mehrzahl von Stents aus Metalllegierungen wie zum Beispiel Edelstahl oder Nitinol hergestellt. Stents können sowohl aus Flachblechen oder Rohren gelasert (US Patent US 4,733,665 A) als auch aus Drähten geflochten oder gewebt werden (US Patent 4,922,905 A). Sind Endoprothesen aus nicht ferromagnetischen Metalllegierungen hergestellt, so können Patienten prinzipiell nach Plazierung von Endoprothesen auch in starken Magnetfeldern untersucht werden, da eine Bewegung der Endoprothesen durch Positionierung im Magnetfeld zum Beispiel eines Magnetresonanz-(MR)-tomographen nicht zu befürchten ist (Shellock FG, Shellock VJ. Metallic stents: evaluation of MR imaging safety. AJR Am J Roentgenol 1999; 173:543-7; Hug J, Nagel E, Bornstedt A, Schnackenburg B, Oswald H, Fleck E. Coronary arterial stents: safety and artifacts during MR imaging. Radiology 2000; 216:781-7). Diese Eigenschaft wird heute gerne als MR-Kompatibilität - genauer als MR-Kompatibilität der ersten Art - bezeichnet(Schenck JF. The role of magnetic susceptibility in magentic resonance imaging: MRI magnetic compatibility of the first and second kinds. Med Phys 1996; 23:815-850). Hierbei wird allerdings nur die fehlende Gefährdung des Patienten berücksichtigt, wenn dieser nach Implantation der MR-kompatiblen Endoprothese einem starken Magnetfeld ausgesetzt wird.

Bisher verwandte Endoprothesen aus Metall, erzeugen Artefakte im Magnetresonanztomographie-Bild, die insbesondere bei kleineren Gefässen eine Beurteilung des Lumens einer Endoprothese mit Hilfe der Magnetresonanztomographie nicht erlauben (Meyer JM, Buecker A, Schuermann K, Ruebben A, Guenther RW. MR evaluation of stent patency: In vitro tests of 22 metallic stents and the possibility of determining their patency by MR angiography. Invest Radiol 2000; 35:739-746; Klemm T, Duda S, Machann J, et al. MR imaging in the presence of vascular stents: A systematic assessment of artifacts for various stent orientations, sequence types, and field strengths. J Magn Reson Imaging 2000; 12:606-15). Dies ist sowohl durch die unterschiedliche Magnetisierbarkeit der verwendeten Metalllegierungen im Vergleich zu menschlichem Gewebe als auch durch Wirbelströme beziehungsweise Radiofrequenzeffekte bedingt (Lüdecke KM, Röschmann P, Tischler R. Susceptibility artifcats in NMR imaging. Magn Reson Imaging 1985; 3:329-343; Camacho CR, Plewes DB, Henkelman RM. Nonsusceptibility artifacts due to metallic objects in MR imaging. J Magn Reson Imaging 1995; 5:75-88). Es gibt allerdings auch einen diagnostischen Ansatz, der unter bestimmten Voraussetzungen den Stent aktiv zur MR-Bildgebung nutzt (US Patent 6,280,385 A1, EP 1023609 B1, WO 99/19738 sowie Quick HH, Ladd ME, Nanz D, Mikolajczyk KP, Debatin JF. Vascular stents as RF antennas for intravascular MR guidance and imaging. Magn Reson Med 1999; 42:738-45).

Die Aufgabe der vorliegenden Erfindung besteht deshalb darin, Endoprothesen herzustellen, die die obigen Nachteile, wie Erzeugung von Artefakten im Magnetresonanztomographie-Bild und eine Gefährdung des Patienten bei der Untersuchung mittels Magnetresonanztomographie auszulösen, nicht aufweisen - und zwar möglichst unabhängig von der verwandten MR-Technik. Es soll dabei sowohl das aussen um die Endoprothese gelegene Gewebe als auch das Lumen dieser Endoprothese mit Hilfe der Magnetresonanztomographiebilder beurteilt werden können. Zusammen mit den fehlenden ferromagnetischen Eigenschaften sollen diese metallischen Endoprothesen als "voll MR-kompatibel" bezeichnet werden können, was der wissenschaftlich beschriebenen Anforderung der MR-Kompatibilität der zweiten Art entspricht.

Diese Aufgabe wird durch eine Endoprothese gemäß Patentanspruch 1-17 gelöst.

Insbesondere handelt es sich um eine Endoprothese aus einem metallischen Material, welches eine magnetische Suszeptibilität im Bereich zwischen -300x10⁻⁶ und 300x10⁻⁶ aufweist, wobei die Endoprothese derart gestaltet ist, daß einzelne Endoprothesenstreben oder -drähte entlang der Endoprothesenlängsachse so orientiert sind, daß diese keinen durchgehenden elektrischen Kreis in einer Ebene, die im wesentlichen senkrecht zur Endoprothesenlängsachse ausgerichtet ist, über die Zirkumferenz der Endoprothese bilden.

Die Erfindung wird weiter anhand der Figuren beschrieben, welche zeigen:
**Fig. 1:**
   Die Zeichnung zeigt ein dreidimensionales Modell eines möglichen voll MR-kompatiblen Designs, das zum Beispiel aus einem Rohr oder Flachblech hergestellt werden kann. Die von einem heilixförmigen Rückgrat ausgehenden Endoprothesenstreben bilden keine geschlossenen Kreise. Es sind einander gegenüberliegende Ösen zur möglichen Befestigung einer Verbindung der Endoprothesenstreben mit dem Rückgrat durch eine nicht oder nur sehr schlecht leitende Struktur eingezeichnet.
**Fig. 2a:**
   Mögliches Design (zweidimensional) einer voll MR-kompatiblen Endoprothese (nicht gedehnt), die zum Beispiel aus einem Rohr oder Flachblech hergestellt werden kann. Die von einem heilixförmigen Rückgrat ausgehenden Endoprothesenstreben bilden keine geschlossenen Kreise. Es sind einander gegenüberliegende Ösen zur möglichen Befestigung einer Verbindung der Endoprothesenstreben mit dem Rückgrat durch eine nicht oder nur sehr schlecht leitende Struktur eingezeichnet.
**Fig. 2b:**
   Mögliches Design (zweidimensional) einer voll MR-kompatiblen Endoprothese (nicht gedehnt), das zum Beispiel aus einem Rohr oder Flachblech hergestellt werden kann. Die von einem helixförmigen Rückgrat ausgehenden Endoprothesenstreben bilden keine geschlossenen Kreise. Es sind einander gegenüberliegende Ösen zur möglichen Befestigung einer Verbindung der Endoprothesenstreben mit dem Rückgrat durch eine nicht oder nur sehr schlecht leitende Struktur eingezeichnet.
**Fig. 2c:**
   Mögliches Design (zweidimensional) einer voll MR-kompatiblen Endoprothese (nicht gedehnt), das zum Beispiel aus einem Rohr oder Flachblech hergestellt werden kann. Die von einem helixförmigen Rückgrat ausgehenden Endoprothesenstreben bilden einmal keine geschlossenen Kreise und einmal einen geschlossenen Kreis. Zur Erhaltung der vollen MR-Kompatibilität müssen die geschlossenen Kreise teilweise aus sehr schlecht oder nicht elektrisch leitenden Zwischenstücken bestehen (1).
**Fig. 2d:**
   Mögliches Design (zweidimensional) einer voll MR-kompatiblen Endoprothese (nicht gedehnt), das zum Beispiel aus einem Rohr oder Flachblech hergestellt werden kann. Die von einem helixförmigen Rückgrat ausgehenden Endoprothesenstreben bilden keine geschlossenen Kreise. Die Endoprothesenstreben bilden jeweils polygonal geformte einander gegenüberliegende Paare.
**Fig. 2e:**
   Mögliches Design (zweidimensional) einer voll MR-kompatiblen Endoprothese (nicht gedehnt), das zum Beispiel aus einem Rohr oder Flachblech hergestellt werden kann. Die von einem helixförmigen Rückgrat ausgehenden polygonalen Endoprothesenstreben bilden keine geschlossenen Kreise. Die Endoprothesenstreben sind sägezahnartig gegeneinander versetzt.
**Fig. 3a, b:**
   Dreidimensonales Modell in Aufsicht (a) und Profilansicht (b), welches die nicht massstabsgetreue Verbindung (dunkelgrau) zwischen einer Endoprothesenstrebe und einem helixförmigen Rückgrat darstellt. Diese Verbindung sollte möglichst flach sein und muss aus einem entweder nicht oder nur sehr schlecht elektrisch leitenden Material bestehen. Die erforderliche minimale Länge des nicht oder nur sehr schlecht elektrisch leitenden Verbindungsstücks hängt davon ab, ob das metallische Material elektrisch isoliert ist oder nicht.
**Fig. 4a - g:**
   Dreidimensionale Schemazeichnungen einer voll MR-kompatiblen Endoprothese (nicht massstabsgetreu), die zum Beispiel aus einem Rohr oder Flachblech hergestellt werden kann. Die Endoprothesenstreben gehen beispielhaft von einem einzelnen geraden Rückgrat aus. Die Endoprothesenstreben können sägezahnartig versetzt und abwechselnd von der einen und anderen Seite des Rückgrates gerade abgehen (4a, b, c) oder aber in einem Winkeln zum Verlauf des Rückgrates (4d). Vorteilhafterweise bilden die Endoprothesenstreben Schlaufen, die wiederum abwechselnd von der einen oder anderen Seite des Rückgrates ihren Ursprung nehmen (4e, f). Die Form der Endoprothesenschlaufen ist vorteilhaft polygonal (4g).
**Fig. 5:**
   Schemazeichnungen, die beispielhaft mögliche Formen des Rückgrates (5a, b) oder der Rückgrate (5c, d) der voll MR-kompatiblen Endoprothese demonstrieren. Vorteilhafterweise hat das Rückgrat eine Helixform (5a, b). Bei Verwendung mehrerer Rückgrate kann die Heilixform für alle Rückgrate beibehalten werden, wobei möglichst wenig Kreuzungsstellen der Rückgrate vorliegen und diese möglichst weit auseinander liegen sollten (5c, d).
**Fig. 6:**
   Magnetresonanztomographie-Bilder von Stents, die in einem Wasserbad positioniert und dann gemessen wurden. Die Orientierung der Stents ist senkrecht zur Magnethauptfeldachse, um die grössten Artefakte zu provozieren.
**Fig. 6a:**
   Geflochtene Stents aus Gold mit Isolierung (1), aus Kupfer ohne Isolierung (2) und aus Kupfer mit Isolierung (3). Der isolierte Kupferstent ist nahezu unsichtbar (3) und der isolierte Goldstent zeigt keine wesentlichen Artefakte, die über die Endoprothesenwand hinausgehen (1). Die Wichtigkeit der Isolierung wird anhand des grossen Artefaktes des nicht isolierten Kupferstents deutlich (2), da Kupfer aufgrund seiner dem menschlichen Gewebe nahezu identischen Suszeptibilität keine Suszeptibilitätsartefakte verursacht.
**Fig. 6b:**
   Gewebte Stents mit verschiedenen Durchmessern aus einer Palladium-Silber-Legierung mit Isolierung, die bei kleinen Durchmessern geringe aber noch akzeptable Artefakte zeigen.
**Fig. 6c:**
   Gelaserte Stents aus einer Kupfer-Gold-Legierung mit (1-4) und ohne (5) geschlossene elektrisch leitende Struktur über die gesamte Zirkumferenz der Endoprothese. Alle Endoprothesen mit der geschlossenen leitenden Struktur nahezu senkrecht auf die Längsachsen der Endoprothesen zeigen im direkten Vergleich zu einer Endoprothese ohne geschlossene leitende Strukur (5) ausgeprägte Artefakte.
**Fig. 6d:**
   Quer geflochtene Stents (1-3) und überwiegend längs geflochtener Stent (4) jeweils aus Kupfer. Alle quer geflochtenen Stents zeigen deutliche Artfakte (1 - 3), Während der längs geflochten Stent (4) artefaktfrei und nahezu unsichtbar im MR-Bild ist, was die Bedeutung des richtigen Designs für die MR-Kompatibilität belegt.
**Fig. 7:**
   Magnetresonanztomographie-Bilder von Schweinen nach Stentplazierung, die mit einer Koronarangiographie-MR-Sequenz aufgenommen wurden.
**Fig. 7a:**
   Zwei in der links deszendierenden Koronararterie (LAD) plazierten Edelstahlstents zeigen ausgeprägte Artefakte, die weder eine Beurteilung des Stentlumens noch der Umgebung der Koronarie in Stentnähe erlauben.
**Fig. 7b:**
   Ein unmittelbar hinter dem gut sichtbaren Abgang der Sinusknotenarterie in der rechten Kranzarterie plazierter geflochtener Stent ist auf dem MR-Bild nicht zu erkennen, was die artefaktfreie Beurteilung sowohl des Stentlumens als auch der -umgebung erlaubt.
**Fig. 7c:**
   Ein im proximalen Bereich des LAD plazierter überwiegend entlang der Stentlängsachse gewebter Stent ist auf dem MR-Bild nicht zu erkennen, was die artefaktfreie Beurteilung sowohl des Stentlumens als auch der -umgebung erlaubt.
**Fig. 7d:**
   Ein im proximalen Bereich des LAD plazierter gelaserter Stent ohne geschlossene elektrisch leitende Kreise ist auf dem MR-Bild nicht zu erkennen, was die artefaktfreie Beurteilung sowohl des Stentlumens als auch der -umgebung erlaubt.
**Fig. 8:**
   Nierenarterien nach Plazierung MR-kompatibler Stents in der rechten und linken Nierenartiere eines Schweins und Darstellung mit Hilfe verschiedener MR-Angiographie(MRA)-Techniken: Spinlabelling (a), Phasenkontrastangiographie vor (b) und nach (c) Stentung, Kontrastmittel-angehobene T1-Gradientenechosequenz (d). Die Phasenkontrastangiographien wurden zum direkten Vergleich vor und nach Stentung durchgeführt. Auf keinem der MR-Bilder ist ein Artefakt zu erkennen, der das Bild stören würde oder auch nur eine Lokalisation der Stents erlaubt. Die Röntgenangiographie nach Kontrastmittelgabe zeigt die Position der Stents in den Nierenartieren (e, Pfeile).

Bei der Bilderzeugung in der Kernspintomographie werden Magnetfelder von 0,064 bis 3 Tesla und teils auch darüber angewendet. Wichtig ist in diesem Zusammenhang insbesondere die Darstellung der arteriellen und venösen Gefässe sowie die Abbildung der Gallenwege, die sich in der klinischen Anwendung etabliert haben. Befinden sich Materialien unterschiedlicher Magnetisierbarkeit (magnetische Suszeptibilität) in unmittelbarer Nachbarschaft, kommt es zu sogenannten Suszeptibilitätsartefakten. Diese führen im MR-Bild zu Signalauslöschungen und Verzerrungen, die eine Beurteilung in diesem Bereich des MR-Bildes unmöglich machen.

Von den Erfindern wurde erkannt, daß zur Vermeidung zu grosser Artefakte bei Endoprothesen diese aus Materialien hergestellt werden sollten, die eine dem menschlichen Gewebe ähnliche Magnetisierbarkeit (magnetische Suszeptibilität) aufweisen. Es haben sich zum Beispiel Kupfer, Gold, Kuper-Gold-Legierungen und Palladium-Silber-Legierungen als geeignet erwiesen, wenn zusätzlich die unten beschriebenen Voraussetzungen für ein voll MR-kompatibles Design eingehalten werden. Neben Suszeptibilitätsartefakten können allerdings trotzdem noch Artefakte durch die Bildung von Wirbelströmen und durch Radiofrequenzeffekte wie beispielsweise die Abschirmung des Inneren einer Endoprothese auftreten.

Von den Erfindern wurde nun im Rahmen der vorliegenden Erfindung erkannt, daß die Kombination von Metallen oder Metalllegierungen ohne wesentlichen Suszeptibilitätsunterschied zu menschlichem Gewebe mit den speziellen Designs einer Endoprothese im wesentlichen das Auftreten von irgendwelchen Artefakten im MR-Bild verhindert. Zur Vermeidung des artefakterzeugenden Flusses von Wirbelströmen beziehungsweise einer Radiofrequenzabschirmung sollte die Möglichkeit eines komplett kreisenden Stromflusses, insbesondere in einer Ebene, die im wesentlichen senkrecht zur Längsachse der Endoprothese ausgerichtet ist, verhindert werden.

Eine erfindungsgemäße Endoprothese kann durch jede dem Fachmann bekannte Herstellungsart hergestellt werden. Geeignete Herstellungsverfahren sind in US Patent US 4,733,665 A, US Patent 4,922,905 A und Palmaz, Cardiovasc. Intervent. Radiol. 1992, 15:279-284 beschrieben, wobei diese Fundstellen durch Bezugnahme hier inkorporiert werden. Es hat sich als vorteilhaft herausgestellt, daß neben geflochtenen oder gewebten Drähten Flachbleche oder Rohre gelasert werden, um Endoprothesen herzustellen. Unabhängig von der Herstellungsart (Laserung versus Flechten/Weben) sollte ein Material verwendet werden, welches keine oder nur minimale Suszeptibilitätsartefakte erzeugt.

Insbesondere sind Implantate mit diesen Eigenschaften für den Einsatz in menschlichen oder tierischen Gefässen, Gefässbypässen, Uretern, intrahepatischen Bypässen, Gallenwegen sowie für den Einsatz in sonstigen Hohlorganen geeignet.

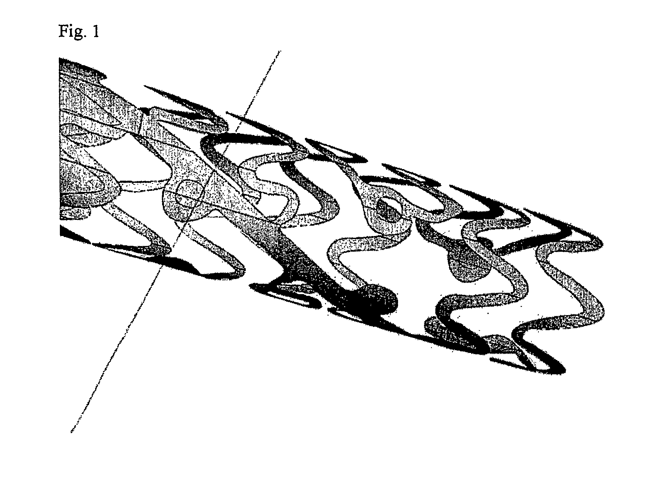

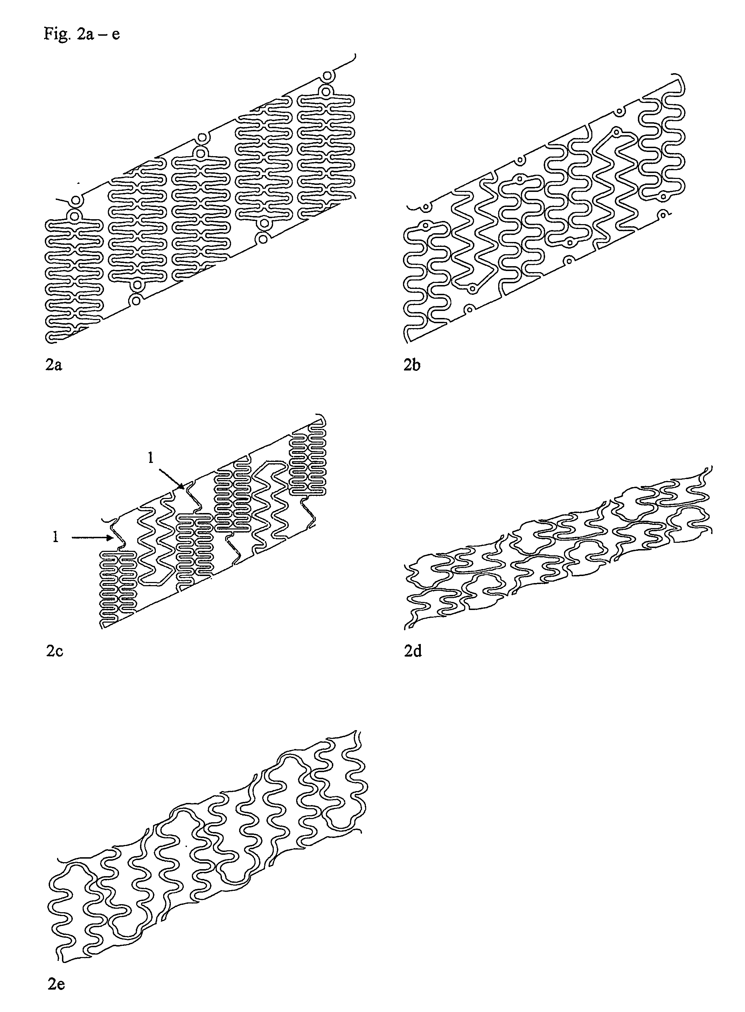

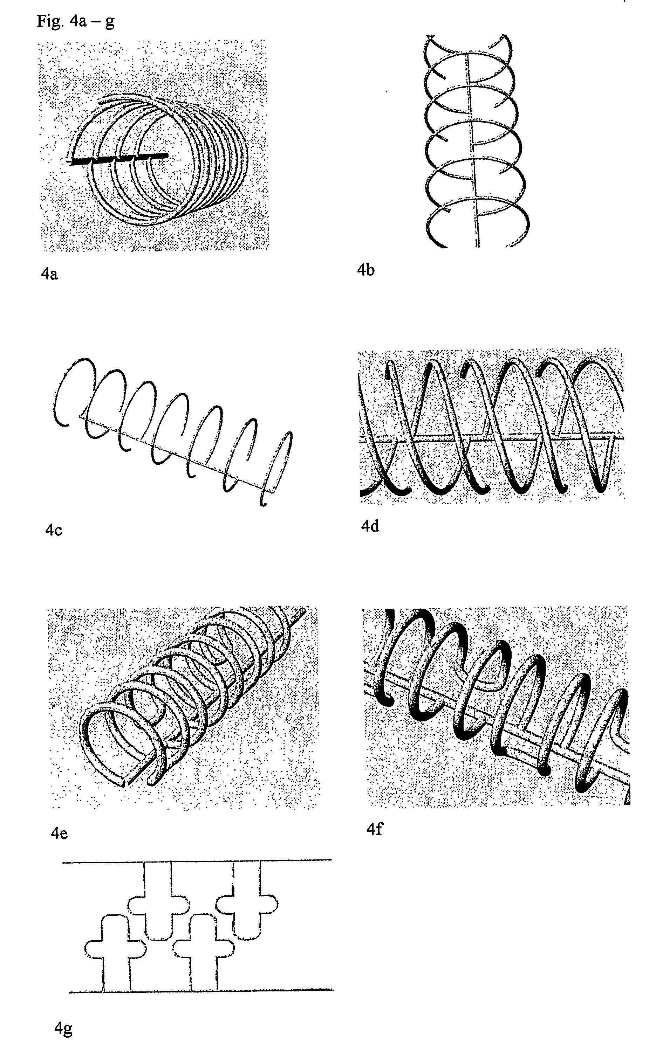

Eine bevorzugte Herstellungsart der erfindungsgemäßen Endoprothesen ist die Laserung, die hinsichtlich der MR-Kompatibilität im nachfolgenden näher beschrieben wird. Im Falle der Laserung der Endoprothesen kommen verschiedene Endoprothesendesigns in Betracht. Es hat sich als besonders vorteilhaft herausgestellt, daß von einem oder mehreren Rückgraten die einzelnen Endoprothesenstreben ausgehen, ohne dass sie beziehungsweise die metallischen Teile dieser Prothesenstreben einen durchgehenden leitenden Kreis in einer Ebene im wesentlichen senkrecht zur Endoprothesenlängsachse über die gesamte Zirkumferenz der Endoprothese bilden können. Hierdurch wird erstens der Aufbau von lokalen Magnetfeldern durch Wirbelströme verhindert und zweitens das Abschirmen des Inneren der Prothese gegen die im Rahmen der MR-Bildgebung eingestrahlten Radiofrequenzenergie erreicht. Das oder die Rückgrate können gerade sein oder eine beliebige Form haben, wobei vor allem eine Helix vorteilhaft ist (Fig. 1, 2, 3). Die Endoprothesenstreben können eine beliebige Form haben, was einzelne stabartige oder geschwungen Streben und auch halbkreisförmig angeordnete gerade oder geschwungene (gekrümmte) Doppelstreben einschliesst (Fig. 2a-e, 4a-g). Die Krümmung oder Biegung von Einzel- oder Doppelstreben kann dabei, vorteilhafterweise unter Berücksichtigung der oben gemachten Voraussetzungen, jede Form annehmen. Einzel- und Doppelstreben können auch kombiniert verwandt werden. So erzeugt die Endoprothese im MR-Bild keine nennenswerten Artefakte, was vor allem auch die Beurteilung des Inneren der Endoprothese mit Hilfe der Magnetresonanztomographie erlaubt. In einer bevorzugten Ausführungsform sind zwischen den Endoprothesenstreben Verbindungsstreben angebracht, die nicht oder nur gering strom-leitend sind.

Eine weitere bevorzugte Herstellungsart ist das Flechten oder Weben, was hinsichtlich der MR-Kompatibilität im nachfolgenden näher beschrieben wird. Soll die Endoprothese aus einem Draht geflochten oder gewebt werden, so sollten ebenfalls die auftretenden Wirbelströme soweit reduziert beziehungsweise so gelenkt werden, dass keine störenden Magnetfelder auftreten oder Radiofrequenzabschirmungen erfolgen. Hierzu werden vorteilhafterweise zum einen die Drähte so isoliert, dass an den Berührungsstellen der Drähte keine leitenden Verbindungen entstehen und zum anderen wird jeder einzelne Draht möglichst in Längsachse der Endoprothese ausgerichtet, so dass vor allem kein geschlossener oder nahezu geschlossener Kreis in einer Ebene im wesentlichen senkrecht zur Längsachse der Endoprothese und über deren gesamte Zirkumferenz entsteht. Dieses Prinzip ist unabhängig davon, ob nur ein einzelner Draht oder mehrere Drähte zur Herstellung der Endoprothese benutzt werden. Ebenso spielt es keine Rolle, ob die Anordnung der Drähte durch Flechten oder Weben erreicht wird. Der Draht oder die Drähte können hierbei Zickzack-, Omega-, Sinus- oder sonstige polygonale Formen annehmen, solange die Hauptausrichtung entlang der Endoprothesenlängsachse erfolgt.

Erfindungsgemäß handelt sich um Endoprothesen, die aus verschiedenen metallischen magnetresonanzkompatiblen Materialien hergestellt werden können. Diese Materialien sind Metalle oder Metalllegierungen, die sich dadurch auszeichnen, dass aufgrund einer dem menschlichen Gewebe ähnlichen Magnetisierbarkeit keine wesentlichen Suszeptibilitätsartefakte in MR-Bildern erzeugt werden. Bei diesen Legierungen handelt es sich bevorzugt um kupfer-, silber-, palladium- oder goldhaltige Metallmischungen. Zusätzlich sind auch die Reinsubstanzen und hierbei insbesondere Kupfer als Herstellungsmaterial der Endoprothesen geeignet. Da das Ausmass möglicher Suszeptibilitätsartefakte neben der Differenz der Magnetisierbarkeiten zweier Substanzen noch von weiteren Faktoren abhängt und minimale Artefakte im MR-Bild tolerierbar sind, können keine festen absoluten Grenzwerte angegeben werden. Die magnetische Suszeptibilität sollte erfindungsgemäß Werte zwischen -300x10⁻⁶ und 300x10⁻⁶ (Werte auf der Basis des MKS(Meter, Kilogram, Sekunde)-Systems einheitslos) haben. Vorteilhafterweise sollte die Suszeptibilität zwischen - 100x10⁻⁶ und 100x10⁻⁶ , ganz bevorzugt zwischen -50x10⁻⁶ und 40x10⁻⁶ , noch mehr bevorzugt zwischen -20x10⁻⁶ und 10x10⁻⁶ liegen. Als zusätzliche beeinflussende Faktoren sind vor allem die Magnetfeldstärke (magnetische Flussdichte) des Magnetresonanztomographen und MR-Sequenzparameter, wie zum Beispiel die Anregungswinkel, Echozeit und Auslesebandbreite, zu nennen. Auch die Orientierung einer Endoprothese zum Magnethauptfeld eines Kernspintomographen spielt eine Rolle für die Grösse eines eventuell auftretenden Suszeptibilitätsartefaktes. Im folgenden ist ein Beispiel von vielen möglichen für die Auswahl einer Metalllegierung angegeben, die die Voraussetzungen für die Herstellung einer voll MR-kompatiblen Endoprothese erfüllt (Angaben in Massenprozent):
- Au: 20,0-80,0%, alternativ 30,0-60,0%, weiter alternativ 30-40%
- Cu: 20,0-80,0%, alternativ 30,0-60,0%, weiter alternativ 50-60%
- Pt: 0-7,5%, alternativ 1-5%, weiter alternativ 1-3%
- Pd: 0-10%, alternativ 1-7,5%, weiter alternativ 1-4%
- Ir: 0-5%, alternativ 0-4%, weiter alternativ 0-2%
- Ag: 0-20%, alternativ 1-10%, weiter alternativ 5-10%
- Zn: 0-5%, alternativ 0-4%, weiter alternativ 0-2%
- Sn: 0-5%, alternativ 0-4%, weiter alternativ 0-2%
- Ru: 0-5%, alternativ 0-4%, weiter alternativ 0-2%
weitere Substanzen insgesamt unter 15%, bevorzugt unter 10%.

Bei den weiteren Substanzen handelt es sich z.B. um Wismut, Antimon, Indium, Thallium, Gold, Quecksilber, Beryllium, Silber, Gallium, Zinn, Kohlenstoff, Phosphor, Selen, Aluminium, Aluminiumoxid, Silicium, Siliciumoxid, Blei, Zink, Schwefel, Magnesiumoxid, Magnesium, Zirkoniumoxid, Zirkonium, Germanium, Silikon, Rubidium, Cesium, Magnesium, Yttrium, Yttriumoxid, Wolfram, Molybdän, Rhodium, Tantal, Titan, Niob, Platin, Vanadium oder Palladium. Die Auswahl dieser Substanzen wurde aufgrund der ihnen eigenen Suszeptibilität getroffen, die nach Erfahrungen der Erfinder im geeigneten Bereich liegt. Hierbei sei ausdrücklich darauf hingewiesen, dass es sich bei diesen Substanzen nicht um eine komplette Liste aller in Betracht kommender Substanzen handelt.

Erfindungsgemäß bevorzugte Endoprothesen weisen die folgenden Zusammensetzungen auf:
z.B.: 35% Au, 54,4% Cu, 2,2% Pt, 1% Pd, 6,75% Ag, 0,6% Sn, 0,05% Ir
oder: 10% Ag, 90% Cu
oder: 50% Ag, 50% Cu
oder: 10% Ni, 90% Cu
oder: 5% Sn, 95% Cu
oder: 60% Pd, 40% Ag.

Allerdings ist das Wirkungsprinzip prinzipiell auch bei Verwendung der Reinsubstanzen funktionstüchtig, wie Versuche mit Cu und Au gezeigt haben. Des weiteren sind alle Metalle und Metalllegierungen, die eine zu menschlichem Gewebe ähnliche magnetische Suszeptibilität aufweisen, als Material zum Bau der vorgestellten Endoprothese geeignet. Dies sind beispielsweise: Kupfer, Gold, Kupfer-Gold-Legierungen, Silber-PalladiumLegierungen.

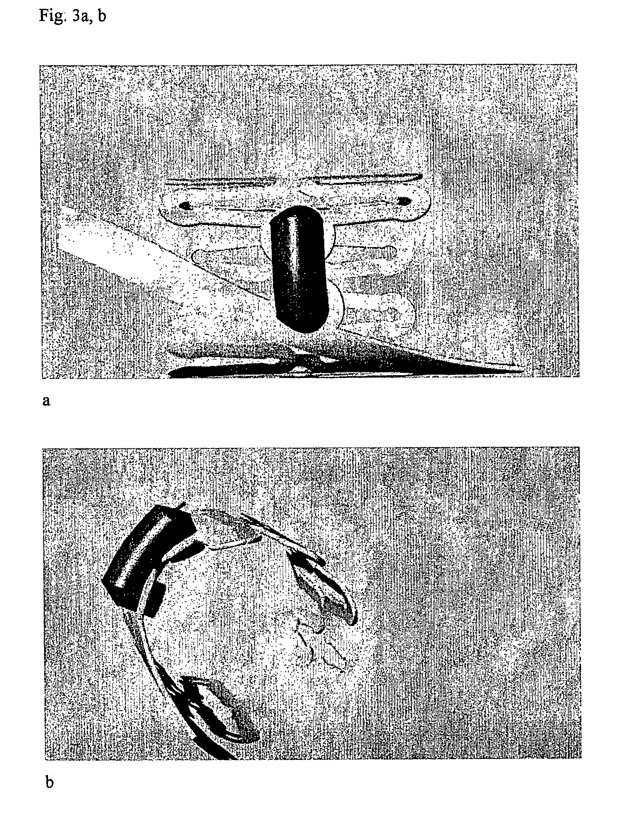

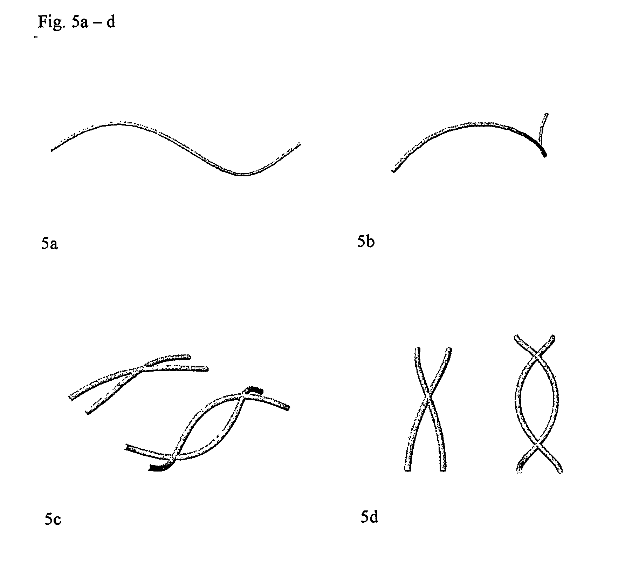

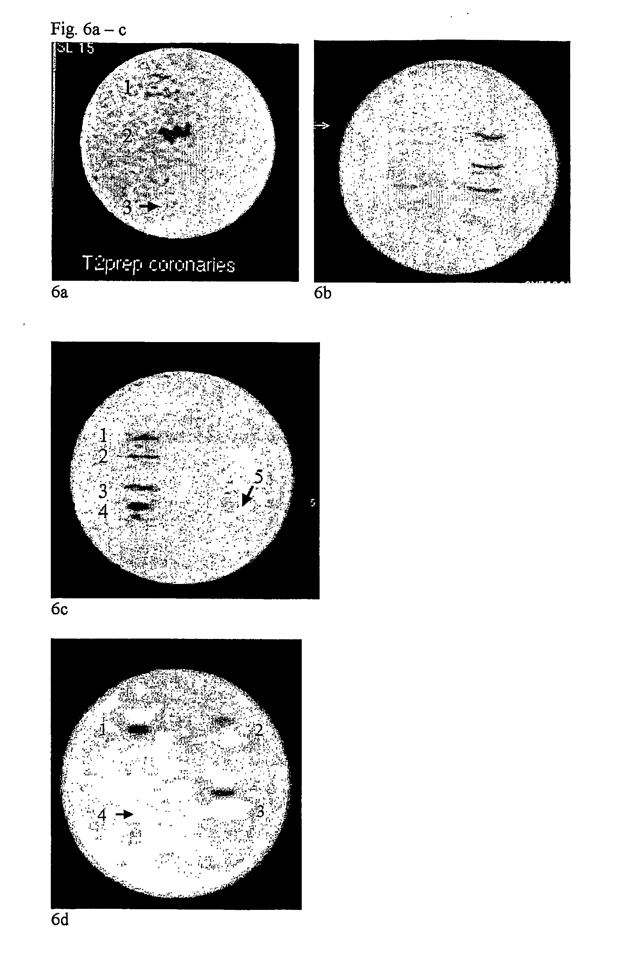

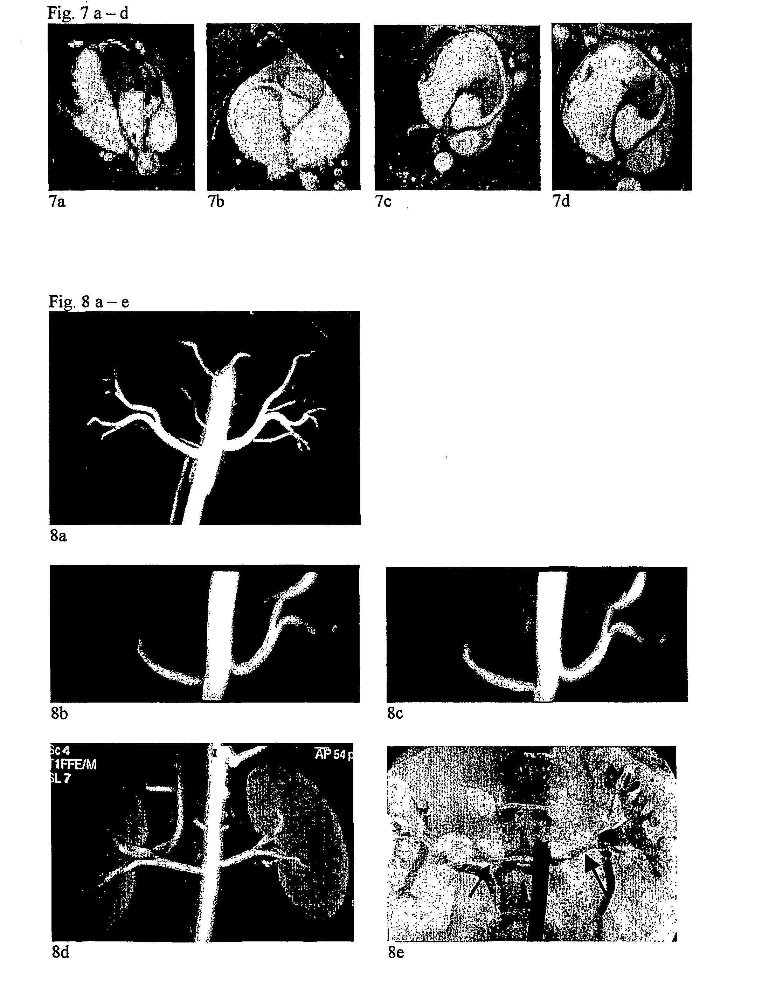

Wird die Endoprothese aus einem Rohr oder Flachblech hergestellt - was im Regelfall vorteilhaft durch Laserung erreicht wird - sollte das Endoprothesendesign so gewählt werden, dass nach Aufdehnung der Endoprothese möglichst kein kreisender Stromfluss entstehen kann, der das Innere der Endoprothese abschirmen könnte. Insbesondere ist die Bildung von geschlossenen Kreisstrukturen über die gesamte Zirkumferenz der Endoprothese durch die Endoprothesenstreben und eines entsprechenden Stromflusses in einer Ebene senkrecht oder nahezu senkrecht zur Endoprothesenlängsachse zu vermeiden. Hierzu werden die einzelnen Endoprothesenstreben nicht kreisförmig in einer Ebene im wesntlichen senkrecht zur Endoprothesenlängsachse zusammengeführt, sondern versetzt oder unmittelbar einander gegenüber liegend angeordnet, ohne jedoch eine durchgehende elektrische Verbindung zueinander aufzuweisen. Die Endoprothesenstreben können zueinander parallel, senkrecht oder in beliebigen Winkeln angeordnet sein (Fig. 2a-e, 4a-d) mit beliebiger Form der Endoprothesenstreben vom Rückgrat oder den Rückgraten ausgehend. Die Endoprothesenstreben können als einzelne Strebe oder als geschlossene oder offene schlaufenartige beziehungsweise polygonale Struktur aus einem oder mehreren Segmenten geformt sein (Fig. 2a-e, 4a-g), ohne jedoch einen kompletten Kreis um die gesamte Zirkumferenz in einer Ebene im wesentlichen senkrecht auf die Längsachse der Endoprothese zu bilden. Im Falle der Bildung einer polygonalen Struktur können die Streben so geformt sein, dass abgerundete Winkel entstehen. Das Design kann so gewählt werden, dass sich diese Endoprothesenstreben senkrecht oder/und parallel zur Endoprothesenlängsachse aufdehnen. Zur Verbesserung der Radialkraft oder zur Verbesserung der gleichmässigen Aufdehnbarkeit können beliebig zwischen den Anteilen der Endoprothese angeordnete und beliebig geformte nicht elektrisch leitende oder nur sehr schlecht leitende zusätzliche Verbindungsstreben angebracht werden (Fig. 3). Diese können durch Isolation der Streben mit z.B. Polytetrafluorethylen (PTFE), Polyethylen, Polyamid, Polyparaxylylen, Polyurethan, isolierenden Poly- oder Monomeren hergestellt werden. Diese können durch Verschweissen, Verkleben, Verknoten oder eine beliebige andere Technik mit der metallischen Struktur der Endoprothese verbunden werden, jeweils ohne dass die volle MR-Kompatibilität hierdurch eingeschränkt wird. Zur Verbindung der einzelnen Endoprothesenstreben miteinander ist ein Rückgrat entlang der Endoprothesenlängsachse notwendig. Dieses kann gerade (Fig. 4) oder geschwungen (Fig. 1, 2, 3, 5) oder polygonal verlaufen, ohne dass die MR-Kompatibilität der Endoprothese beeinträchtigt wird. Die Endoprothesen können entweder ein oder auch mehrere solcher Rückgrate besitzen, die entweder gerade sein oder eine beliebige Form haben, wobei eine Helix vorteilhaft ist (Fig. 1 - 3, 5). Werden mehrere Rückgrate verwandt, so sind diese bevorzugt mit nur einer Kreuzungsstelle (Fig. 5c, d) oder möglichst weit auseinander liegenden Kreuzungsstellen anzuordnen. Senkrecht zur Längsachse der Endoprothese verlaufende Kreise sind auch bei der Verbindung der Rückgrate möglichst zu vermeiden. Dementsprechend sollten zum Beispiel bei zwei sinusförmig verlaufenden Rückgraten die Verbindung dieser beiden Rückgrate nur bei geraden oder ungeraden Vielfachen von 90° erfolgen (Fig. 5c, d). Je nach angestrebter Radialkraft und Bedeckungsfläche durch die Endoprothese können die einzelnen Rückgrate mit den verschiedenen oben beschriebenen Formen an Endoprothesenstreben versehen werden.

Wird die Endoprothese nicht aus einem Flachblech oder Rohr, sondern aus einem Draht oder mehreren Drähten hergestellt, so werden die Drähte mit einer elektrischen Isolation versehen. Prinzipiell ist hier die Verwendung einer biokompatiblen, elektrisch nicht leitenden oder geringfügig strom-leitenden Beschichtung erstrebenswert. Bevorzugt ist die Isolierung an den Berühungsstellen und betrifft ganz bevorzugt über 80% des Drahtes. Bevorzugte Materialien zur Isolation sind Kunststoffe wie Polytetrafluorethylen (PTFE), Polyethylen, Polyamid, Polyparaxylylen, Polyurethan, isolierende Poly- oder Monomere. Weitgehend geschlossene Kreise senkrecht zur Längsachse der Endoprothese sind zu vermeiden, was durch eine weitgehende Orientierung des Drahtes beziehungsweise der Drähte entlang der Endoprothesenlängsachse erreicht wird. Für das Erreichen und die Gewährleistung der vollen MR-Kompatibilität ist es hierbei unerheblich, ob die Endoprothese durch Flechten oder Weben oder aus wievielen einzelnen Drähten sie hergestellt wird.

Die Endoprothesen können einfach oder mehrfach mit einer oder mehreren Substanzen innen und/oder außen beschichtet sein, die teils oder insgesamt als Substanz medizinisch oder auch nicht medizinisch wirksam sein können und die entweder dauerhaft gebunden sind und/oder über die Zeit abgegeben werden. Die Beschichtungen können zum Beispiel aus fettlöslichen Vitaminen A, D, E, K, und deren Derivaten, Kortison und seinen Derivaten, Heparin und seinen Derivaten, Immunsuppressiva oder Chemotherapeutika bestehen. Die Endoprothese kann auch mit einer Ummantelung mit einer oder mehreren Membranen inner- und/oder ausserhalb der Endoprothese versehen werden. Als Ummantelungsmaterialien sind beispielsweise PTFE, Polyurethan oder Polyester zu nennen. Die Beschichtungen oder Ummantelungen an der Innen- oder/und Aussenseite haben jeweils keinen Einfluss auf die volle MR-Kompatibilität. Zur Verbesserung der allgemeinen Stenteigenschaften können derartige Modifikationen also vorgenommen werden, ohne dass es hierdurch zu einer Beeinträchtigung der vollen MR-Kompatibilität der Endoprothesen kommt. Die Endoprothesen können auch mit Markern zur besseren Sichtbarmachung unter Röntgendurchleuchtung oder / und in der Magnetresonanztomographie versehen sein. Beispiele füt solche Marker sind Goldringe oder Ringe aus Lanthaniden oder kleinste Eisenpartikel.

## Patentansprüche

1. Endoprothese aus einem metallischen Material, welches eine magnetische Suszeptibilität im Bereich zwischen -300x10⁻⁶ und 300x10⁻⁶ aufweist, wobei die Endoprothese eine Endoprothesenlängsachse und eine Zirkumferenz aufweist und derart gestaltet ist, daß einzelne Endoprothesenstreben oder -drähte entlang der Endoprothesenlängsachse so orientiert sind, daß diese keinen durchgehenden elektrischen Kreis in einer Ebene, die im wesentlichen senkrecht zur Endoprothesenlängsachse ausgerichtet ist, über die Zirkumferenz der Endoprothese bilden.

2. Endoprothese nach Patentanspruch 1, wobei diese aus einem Flachblech oder Rohr hergestellt ist.

3. Endoprothese nach Patentanspruch 2, wobei von einem oder mehreren Rückgraten aus einzelne Endoprothesenstreben ausgehen.

4. Endoprothese nach Patentanspruch 2, wobei das oder die Rückgrate gerade sind.

5. Endoprothese nach Patentanspruch 2, wobei das oder die Rückgrate helixförmig sind.

6. Endoprothese nach Patentanspruch 2, 3, 4 oder 5, wobei die Endoprothesenstreben als einzelne Strebe oder als geschlossene oder offene polygonale Struktur geformt ist

7. Endoprothese nach Anspruch 6, wobei diese ein oder mehrere Segmente aufweist.

8. Endoprothese nach Anspruch 6 oder 7, wobei Endoprothesenstreben senkrecht oder in beliebigem Winkel von dem oder den Rückgraten ausgehen.

9. Endoprothese nach Patentanspruch 1, wobei diese aus einem oder mehreren isolierten Drähten hergestellt ist, die weitgehend entlang der Längsachse orientiert sind.

10. Endoprothese nach Patentanspruch 1, wobei die Drähte mindestens an den Berührungsstellen elektrisch isoliert sind.

11. Endoprothese nach Patentanspruch 1, wobei mindestens 80% des Drahtes bzw. der Drähte elektrisch isoliert ist.

12. Endoprothese nach einem der Patentansprüche 2 - 8, wobei zwischen den Endoprothesenstreben Verbindungsstreben aus einem isolierenden Material angebracht sind.

13. Endoprothese nach einem der Patentansprüche 2 - 8, wobei zwischen den Endoprothesenstreben Verbindungsstreben aus einem gering strom-leitenden Material angebracht sind.

14. Endoprothese nach einem der Patentansprüche 1-10, wobei das metallische Material umfaßt:
Au 20,0-80,0%
Cu 20,0-80,0%
Pt 0-7,5%
Pd 0-10%
Ir 0-5%
Ag 0-20%
Zn 0-5%
Sn 0-5%
Ru 0-5%
weitere Substanzen insgesamt 0 bis weniger als 15%.

15. Endoprothese nach Anspruch 14, wobei die weiteren Substanzen ausgewählt sind aus Wismut, Antimon, Indium, Thallium, Gold, Quecksilber, Berylium, Silber, Gallium, Zinn, Kohlenstoff, Phosphor, Selen, Aluminium, Aluminiumoxid, Silicium, Siliciumoxid, Blei, Zink, Schwefel, Magnesiumoxid, Magnesium, Zirkoniumoxid, Zirkonium, Germanium, Silikon, Rubidium, Cesium, Magnesium, Yttrium, Yttriumoxid, Wolfram, Molybdän, Rhodium, Tantalum, Titan, Niobium, Platin, Vanadium oder Palladium .

16. Endoprothese nach einem der Patentansprüche 1-15, wobei die Endoprothese einfach oder mehrfach beschichtet ist.

17. Endoprothese nach einem der Patentansprüche 1-16, wobei eine der Beschichtungen die fettlöslichen Vitaminen A, D, E, K und deren Derivate, Kortison und seine Derivate, Heparin und seine Derivate, Immunsuppressiva oder Chemotherapeutika enthält.

18. Endoprothese nach einem der Patentansprüche 1-16, wobei die Endoprothese mit einer Ummantelung aus einer oder mehreren Membranen umgeben ist.

## Claims

1. An endoprosthesis comprising a metallic material having a magnetic susceptibility in the range of between -300 x 10⁻⁶ and 300 x 10⁻⁶, wherein the endoprosthesis has an endoprosthesis longitudinal axis and a circumference and is of such a configuration that individual endoprosthesis bars or wires are so oriented along the endoprosthesis longitudinal axis that they form no continuous electrical circuit in a plane which is oriented substantially perpendicularly to the longitudinal axis of the endoprosthesis, over the circumference of the endoprosthesis.

2. An endoprosthesis according to claim 1 wherein it is produced from a flat sheet or tube.

3. An endoprosthesis according to claim 2 wherein individual endoprosthesis bars extend from one or more backbones.

4. An endoprosthesis according to claim 2 wherein the backbone or backbones are straight.

5. An endoprosthesis according to claim 2 wherein the backbone or backbones are helical.

6. An endoprosthesis according to claim 2, claim 3, claim 4 or claim 5 wherein the endoprosthesis bars are formed as individual bars or are in the form of a closed or open polygonal structure.

7. An endoprosthesis according to claim 6 wherein it has one or more segments.

8. An endoprosthesis according to claim 6 or claim 7 wherein endoprosthesis bars extend perpendicularly or at any angle from the backbone or backbones.

9. An endoprosthesis according to claim 1 wherein it is produced from one or more insulated wires which are oriented largely along the longitudinal axis.

10. An endoprosthesis according to claim 1 wherein the wires are electrically insulated at least at the contact locations.

11. An endoprosthesis according to claim 1 wherein at least 80% of the wire or wires is electrically insulated.

12. An endoprosthesis according to one of claims 2 to 8 wherein connecting bars of a insulating material are disposed between the endoprosthesis bars.

13. An endoprosthesis according to one of claims 2 to 8 wherein connecting bars of a slightly current-conducting material are disposed between the endoprosthesis bars.

14. An endoprosthesis according to one of claims 1 to 10 wherein the metallic material includes:
Au 20.0-80.0%
Cu 20.0-80.0%
Pt 0-7.5%
Pd 0-10%
Ir 0-5%
Ag 0-20%
Zn 0-5%
Sn 0-5%
Ru 0-5%
further substances a total of 0 to less than 15%.

15. An endoprosthesis according to claim 14 wherein the further substances are selected from bismuth, antimony, indium, thallium, gold, mercury, beryllium, silver, gallium, tin, carbon, phosphorus, selenium, aluminium, aluminium oxide, silicon, silicon oxide, lead, zinc, sulphur, magnesium oxide, magnesium, zirconium oxide, zirconium, germanium, silicone, rubidium, caesium, magnesium, yttrium, yttrium oxide, tungsten, molybdenum, rhodium, tantalum, titanium, niobium, platinum, vanadium or palladium.

16. An endoprosthesis according to one of claims 1 to 15 wherein the endoprosthesis is singly or multiply coated.

17. An endoprosthesis according to one of claims 1 to 16 wherein one of the coatings contains the fat-soluble vitamins A, D, E and K and derivatives thereof, cortisone and derivatives thereof, heparin and derivatives thereof, immunosuppressives or therapeutic agents.

18. An endoprosthesis according to one of claims 1 to 16 wherein the endoprosthesis is surrounded by a casing comprising one or more membranes.

## Revendications

1. Endoprothèse en matériau métallique, qui présente une susceptibilité magnétique située dans la plage entre - 300 x 10⁻⁶ et 300 x 10⁻⁶, l'endoprothèse présentant un axe longitudinal d'endoprothèse et une circonférence et étant conformée de telle sorte que les armatures ou fils d'endoprothèse individuels sont orientés le long de l'axe longitudinal de l'endoprothèse de sorte qu'ils ne forment sur la circonférence de l'endoprothèse aucun circuit électrique continu dans un plan qui est orienté sensiblement perpendiculairement à l'axe longitudinal de l'endoprothèse.

2. Endoprothèse selon la revendication 1, dans laquelle celle-ci est fabriquée à partir d'une tôle plate ou d'un tube.

3. Endoprothèse selon la revendication 2, dans laquelle les armatures d'endoprothèse individuelles partent d'une ou plusieurs arêtes dorsales.

4. Endoprothèse selon la revendication 2, dans laquelle la ou les arêtes dorsales sont rectilignes.

5. Endoprothèse selon la revendication 2, dans laquelle la ou les arêtes dorsales ont une forme hélicoïdale.

6. Endoprothèse selon la revendication 2, 3, 4 ou 5, les armatures d'endoprothèse sont formées en tant qu'armatures individuelles ou en tant que structure polygonale fermée ou ouverte.

7. Endoprothèse selon la revendication 6, dans laquelle celle-ci présente un ou plusieurs segments.

8. Endoprothèse selon la revendication 6 ou 7, dans laquelle les armatures d'endoprothèse parte perpendiculairement ou selon un angle quelconque de l'arête ou des arêtes dorsales.

9. Endoprothèse selon la revendication 1, dans laquelle celle-ci est fabriquée à partir d'un ou de plusieurs fils isolés, qui sont orientés largement le long de l'axe longitudinal.

10. Endoprothèse selon la revendication 1, dans laquelle les fils sont isolés électriquement au moins aux points de contact.

11. Endoprothèse selon la revendication 1, dans laquelle au moins 80 % du fil ou des fils sont isolés électriquement.

12. Endoprothèse selon l'une des revendications 2 à 8, dans laquelle des armatures de liaison en matériau isolant sont disposées entre les armatures d'endoprothèse.

13. Endoprothèse selon l'une quelconque des revendications 2 à 8, dans laquelle des armatures de liaison en un matériau faiblement électroconducteur sont disposées entre les armatures d'endoprothèse.

14. Endoprothèse selon l'une quelconque des revendications 1 à 10, dans laquelle le matériau métallique comprend :
Au 20,0 - 80,0 %
Cu 20,0 - 80,0 %
Pt 0 - 7,5 %
Pd 0-10%
Ir 0 - 5 %
Ag 0 - 20 %
Zn 0-5 %
Sn 0 - 5 %
Ru 0 - 5 %
ou d'autres substances présentes au total entre 0 et moins de 15 %.

15. Endoprothèse selon la revendication 14, dans laquelle les autres substances sont choisies parmi les suivantes : bismuth, antimoine, indium, thallium, or, mercure, béryllium, argent, gallium, étain, carbone, phosphore, sélénium, aluminium, oxyde d'aluminium, silicium, oxyde de silicium, plomb, zinc, soufre, oxyde de magnésium, magnésium, oxyde de zirconium, zirconium, germanium, silicone, rubidium, césium, magnésium, yttrium, oxyde d'yttrium, tungstène, molybdène, rhodium, tantale, titane, niobium, platine, vanadium ou palladium.

16. Endoprothèse selon l'une des revendications 1 à 15, dans laquelle l'endoprothèse est revêtue de façon simple ou multiple.

17. Endoprothèse selon l'une des revendications 1 à 16, dans laquelle l'un des revêtements contient les vitamines liposolubles A, D, E, K et leurs dérivés, de la cortisone et ses dérivés, de l'héparine et ses dérivés, des produits immunosuppresseurs ou des produits de chimiothérapie.

18. Endoprothèse selon l'une des revendications 1 à 16, dans laquelle l'endoprothèse est entourée d'une enveloppe constituée d'une ou de plusieurs membranes.
